# FASCICULE DE BREVET EUROPEEN

(11) **EP 1 352 630 B1**
(45) Date de publication et mention de la délivrance du brevet: **01.03.2006**
(21) Numéro de dépôt: 03290860.0
(22) Date de dépôt: 07.04.2003
(51) Int. Cl.: A61Q 5/10, A61K 8/19, A61K 8/46, A61Q 5/00

(54) **Procédé de gainage métallique conférant aux fibres kératiniques des propriétés cosmétiques rémanentes aux shampoings**
Metallbildungsverfahren welches keratinischen Fasern shampoo-artige kosmetische Eigenschaften verleiht
Metallic shaping process which imparts cosmetic properties similar to shampooing to keratinous fibres

(30) Priorité: 08.04.2002 FR 2043252
(43) Date de publication de la demande: 15.10.2003
(73) Titulaire: L'OREAL, 75008 Paris (FR)
(72) Inventeur: Vic, Gabin, 60200 Compiegne (FR); Livoreil, Aude, 75006 Paris (FR); Giroud, Franck, 92110 Clichy (FR)
(74) Mandataire: Casalonga, Axel

(56) Documents cités:
- EP-A- 1 064 918
- EP-A- 1 243 249
- DE-A- 2 806 603
- DE-U- 29 621 557
- US-A- 1 055 355
- US-A- 3 194 734
- US-A- 4 195 972
- US-A- 4 971 596
- PATENT ABSTRACTS OF JAPAN vol. 015, no. 243 (C-0842), 21 juin 1991 (1991-06-21) & JP 03 077806 A (KANEBO LTD), 3 avril 1991 (1991-04-03)
- PATENT ABSTRACTS OF JAPAN vol. 016, no. 509 (C-0997), 21 octobre 1992 (1992-10-21) & JP 04 187625 A (KATSUMI MIZUMAKI), 6 juillet 1992 (1992-07-06)

## Description

La présente invention est relative à un procédé de gainage métallique conférant aux fibres kératiniques, et notamment aux cheveux, des propriétés cosmétiques rémanentes aux shampooings.

Il est connu de traiter les fibres kératiniques par adsorption physico-chimique ou dépôt de matière sur la surface des fibres afin d'augmenter leur brillance, de les colorer ou de les rendre plus résistantes à la chaleur à la lumière, etc.

La demande EP-A-1064918 décrit l'application sur les cheveux d'une composition cosmétique contenant des particules de type métallique.

La demande JP-A-03090012 décrit l'application sur la peau et les cheveux d'une composition cosmétique comprenant des protéines teintées avec des particules d'or.

La demande JP-A-03077806 décrit l'application sur la peau et les cheveux d'une composition comprenant une poudre de fibres de soie teintées avec des particules d'or.

La demande JP-A-03077806 décrit l'application sur la peau et les cheveux d'une composition cosmétique comprenant un hydrosol de fines particules d'or.

Le document DE 28 06 603 décrit un procédé de teinture de la barbe, des cils et des sourcils consistant à appliquer sur ces fibres une solution A comprenant de la benzocatéchine et de l'acide ascorbique, puis une solution B comprenant du nitrate d'argent.

Le document US 1,055,355 décrit un produit de teinture des cheveux ou de la barbe constitué de deux couches solides superposées, l'une comprenant de la graisse et un sel d'argent, et l'autre couche comprenant de la graisse et l'acide pyrogallique.

Le document US 4,971,596 décrit un procédé de teinture selon lequel on applique sur les cheveux, dans un ordre quelconque, une composition comprenant un sel métallique, et une composition comprenant une hydroxyquinone.

Le document US 4,195,972 décrit l'application sur les cheveux d'une composition comprenant un sel métallique et un agent réducteur.

Le document US 3,194,734 décrit une composition cosmétique comprenant un dérivé méthylé du dihydroxy-5,6-indole.

Ces documents ne décrivent pas l'application sur les fibres kératiniques, postérieurement au gainage métallique, d'une composition comprenant un composé de type thiol.

Toutefois, ces traitements ont des effets peu rémanents aux shampooings car la matière déposée est rapidement éliminée sous l'action de l'eau et des agents tensio-actifs. Les procédés classiques de traitement cosmétique capillaire confèrent donc peu de rémanence au dépôt sous l'action des shampooings et les effets cosmétiques apportés s'estompent très rapidement.

La présente invention se propose de remédier à ces inconvénients.

En particulier, la Demanderesse vient de découvrir de façon surprenante que l'enrobage de la fibre kératinique par un métal natif suivi de l'application d'un composé particulier permettait de conférer aux fibres kératiniques des propriétés cosmétiques rémanentes aux shampooings.

Ainsi, les fibres kératiniques possédant cet enrobage métallique présentent des propriétés cosmétiques rémanentes aux shampooings, comme la brillance, le reflet, ou la coloration.

La présente invention a donc pour objet un procédé de traitement cosmétique des fibres kératiniques, consistant à former in situ sur les fibres un métal natif par réduction d'un sel métallique en présence d'un agent réducteur, puis à appliquer un composé particulier.

D'autres objets de l'invention apparaîtront à la lecture de la description et des exemples qui suivent.

Le procédé conforme à l'invention est essentiellement caractérisé par le fait qu'il consiste à procéder à la réduction, dans un milieu cosmétiquement acceptable, d'au moins un sel métallique en présence d'au moins un agent réducteur, directement sur les fibres kératiniques pour former in situ le métal natif correspondant, et en ce que postérieurement à la formation du métal natif correspondant, on applique sur les fibres kératiniques une composition comprenant au moins un composé choisi parmi les thiols, les thiolates, les disulfures, les thioéthers, les xanthates, les thiocarbamates, les thiosulfates et les thiolactones.

Selon un mode de réalisation de l'invention, la fibre kératinique est préalablement réduite, avant d'être mise en présence du sel métallique, afin de libérer des groupes SH qui ont la propriété de réagir avec les métaux pour former une liaison S-métal qui augmente la rémanence du métal dans la fibre.

Dans une étape ultérieure, des actifs choisis parmi les thiols, les thiolates, les disulfures, les thioéthers, les xanthates, les thiocarbamates, les thiosulfates et les thiolactones, à condition qu'ils ne réduisent pas les sels métalliques utilisés dans le procédé ci-dessus, sont greffés sur la fibre kératinique gainée par le dépôt métallique obtenu.

Le métal du sel métallique peut être choisi parmi l'aluminium, les métaux alcalins, les métaux alcalinoterreux, les métaux de transition, les métaux de terres rares et leurs alliages. De préférence, le métal est choisi parmi l'aluminium, le cuivre, l'argent, l'or, l'indium, le fer, le platine, le nickel, le molybdène, le titane, le tungstène, l'antimoine, le palladium, le zinc, l'étain et leurs alliages.

Le sel métallique peut être choisi parmi les sels minéraux ou organiques, acides ou basiques tels que les nitrates, sulfates, carbonates, halogénures, bromates, phosphates et sulfonates.

De préférence, le sel métallique est choisi parmi AgNO₃, AgBrO₃, Ag₂SO₄, AgBr, Ag₂CO₃, AgCl, AgF, AgI, HAuCl₄, AuBr₃, AuCl₃, K₂PdCl₄, CuCl, CuBr, CuSO₄ et Na₂PtCl₆. Les sels de métaux nobles, comme HAuCl₄, AgNO₃, K₂PdCl₄, Na₂PtCl₆ sont préférés. On peut également utiliser des mélanges de sels métalliques.

L'agent réducteur peut être choisi parmi les réducteurs organiques ou minéraux tels que NaBH₄, KBH₄, BH₃, NADPH, NADH, H₂, 9-BBN (9 borabicyclo[[3.3.1]nonane), LiAlH₄, NaAlEt₂H₂, Na₂S₂O₃, le diisoamylborane, l'acide ascorbique et les composés cycliques ou aliphatiques contenant le groupe chimique enediol -(OH)C=C(OH)- incluant les formes isomériques, leurs sels et leurs esters, l'hydroquinone, la benzoquinone, NaBH(OAc)₃, l'acide formamidine sulfinique (FASA), l'acide N-phénylformadimine sulfinique, les phosphines, NaBH₃CN, Na₂S₂O₄, BMS (bismercaptoéthylsulfone), DTT (dithiothréitol), DMH (N,N'diméthyl-N,N'-bis(mercaptoacétyl)hydrazine), les thiols, les thiolates, les citrates, sulfites, bisulfites, monopersulfates et bisulfates de métaux alcalins, les enzymes (comme par exemple la protéine disulfure isomérase ou la thioredoxine), et les sulfinates, en particulier les hydroxyméthane sulfinates.

Les sels métalliques et les agents réducteurs sont généralement utilisés indépendamment les uns des autres à une concentration comprise entre 0,0001% et 50%, de préférence comprise entre 0,01% et 10%, et encore de préférence entre 0,05% et 5%, sur la base du milieu cosmétiquement acceptable.

Le milieu cosmétiquement acceptable utilisé conformément à l'invention est constitué de préférence par de l'eau, éventuellement mélangée à un solvant organique choisi dans le groupe comprenant les alcools en C₁-C₄ tels que l'éthanol ou l'isopropanol, les alcanes en C₅-C₁₀, l'acétone, la méthyléthylcétone, l'acétate de méthyle, l'acétate de butyle, l'acétate d'éthyle, le diméthoxyéthane, le diéthoxyéthane et leurs mélanges.

Les compositions utilisées selon l'invention comprenant au moins un sel métallique ou au moins un agent réducteur dans un milieu cosmétiquement acceptable peuvent en outre comprendre des additifs cosmétiques usuels choisis notamment parmi les agents adhésifs, les agents épaississants, les adoucissants, les agents anti-mousse, les filtres solaires, les agents antiperspirants, les agents acidifiants, les agents hydratants, les agents alcalinisants, les colorants, les pigments, les parfums, les conservateurs, les tensioactifs anioniques, cationiques, non ioniques ou amphotères, les polymères fixants, notamment les polymères fixants non ioniques, cationiques, amphotères, les polymères non fixants, les silicones volatiles ou non, les huiles végétales, animales, minérales ou de synthèse, les protéines et les vitamines, les polyols et leurs mélanges.

Les compositions utilisées conformément à l'invention peuvent être conditionnées sous diverses formes, notamment dans un dispositif aérosol, avec un agent propulseur. Elles peuvent être utilisées en application rincée ou non.

De préférence, l'agent propulseur est choisi parmi l'air, le gaz carbonique, l'azote comprimé, le diméthyléther, les alcanes en C₃-C₅, comme le n-butane, le propane, l'isobutane, les hydrocarbures halogénés, comme le 1,1-difluoroéthane, les mélanges de diméthyléther et d'alcanes en C₃-C₅, les mélanges de 1,1-difluoroéthane et de diméthyléther et/ou d'alcanes en C₃-C₅.

Le procédé conforme à l'invention peut être utilisé pour la fixation et/ou le maintien des cheveux, le soin des cheveux, les shampooings, le conditionnement des cheveux, par exemple pour leur apporter de la douceur, ou encore pour le maquillage des cheveux.

Les compositions utilisées selon l'invention peuvent se présenter sous diverses formes galéniques, telles qu'une lotion, un gel, un stick, une crème, une émulsion simple ou multiple, un spray, une mousse, un shampooing ou un après-shampooing.

Le procédé de traitement des fibres kératiniques, notamment des cheveux, selon l'invention, comporte généralement les étapes suivantes :
- on applique sur les fibres kératiniques une composition contenant au moins un sel métallique, dans un milieu cosmétiquement acceptable,
- après un temps de pose, allant de 30 s à 30 mn, de préférence de 1 à 15 mn, sous l'action ou non de la chaleur, et un éventuel rinçage, on applique une composition contenant au moins un agent réducteur, dans un milieu cosmétiquement acceptable,
- on procède au rinçage après un nouveau temps de pose allant de 30 s à 30 mn, de préférence de 1 à 15 mn, sous l'action ou non de la chaleur,
- on applique sur les fibres kératiniques une composition contenant au moins un composé choisi parmi les thiols, les thiolates, les disulfures, les thioéthers, les xanthates, les thiocarbamates, les thiosulfates et les thiolactones.

Le sel métallique ainsi que l'agent réducteur peuvent être choisis parmi ceux précédemment décrits.

Il est à noter que le gainage métallique selon l'invention peut être enlevé des fibres kératiniques, en cas de besoin, par simple traitement avec de l'eau oxygénée à pH acide à une concentration comprise entre 1 et 30 volumes (entre 0,3 et 9% en poids).

Les fibres kératiniques peuvent être préalablement enduites d'un polymère ayant une affinité pour la surface comme les polyamines (PEI, polylysines), les dérivés de polysaccharides (hydroxypropylcellulose, hydroxypropylguar, Jaguar) ou avoir subi un traitement cosmétique classique comme une coloration d'oxydation, une permanente, une décoloration, un shampooing ou une application de produit de coiffage.

Selon un mode de réalisation de l'invention, les fibres kératiniques peuvent être réduites, préalablement à l'application de la composition contenant au moins un sel métallique. Cette réduction peut être effectuée avec l'un des agents réducteurs décrits plus haut et en particulier les agents réducteurs utilisés dans le cadre de la permanente. Un des agents réducteurs préférés est l'acide thioglycolique. Les fibres peuvent ensuite être rincées. Le ou les sels métalliques puis le ou les agents réducteurs sont alors appliqués comme décrit ci-dessus.

Le composé choisi parmi les thiols, les thiolates, les disulfures, les thioéthers, les xanthates, les thiocarbamates, les thiosulfates et les thiolactones, peut porter des substituants à activité cosmétique. Ces substituants peuvent provenir par exemple de polymères naturels ou synthétiques, solubles ou insolubles dans l'eau, de particules organiques ou métalliques, de pigments, de filtres solaires, d'anti-oxydants, de colorants ou d'agents conditionneurs.

Les exemples suivants sont destinés à illustrer l'invention.

### Exemple 1 : gainage métallique d'argent sur cheveux naturels pigmentés, blancs ou légèrement éclaircis, non réduits

Les mèches (1g) sont lavées au shampooing, séchées 20 mn à 65°C et disposées chacune dans une gouttière appropriée. On applique sur chaque mèche 2 ml d'une solution aqueuse de nitrate d'argent (0,46 M) et on laisse poser 5 mn. On applique ensuite sur chaque mèche 1 ml d'une solution aqueuse de borohydrure de sodium (0,92M) et on laisse poser 15 mn. Les mèches sont alors lavées à l'eau et séchées pendant 20 mn à 60°C.

On obtient les résultats visuels suivants :
- les mèches de cheveux naturels pigmentés sont plus noires, avec des reflets brillants métallisés ;
- les mèches de cheveux blancs sont teintées en jaune-doré avec des reflets brillants métallisés ;
- les mèches de cheveux sensibilisés, légèrement éclaircis, sont teintées en cuivré avec des reflets brillants métallisés.

Les mèches sont lavées dix fois avec un shampooing standard et aucune différence notable de coloration n'est observée entre une mèche non lavée et une mèche lavée dix fois. La couleur obtenue est donc rémanente aux shampooings.

La présence d'un gainage d'argent sur la surface du cheveu a été confirmée par microscopie électronique à balayage et analyse par EDX.

### Exemple 2 : gainage métallique d'argent sur cheveux naturels pigmentés, blancs ou légèrement éclaircis, réduits

### Etape 1 : réduction des mèches.

Les mèches (1g) sont disposées chacune dans une gouttière appropriée. On applique sur chaque mèche 2,4 ml d'une solution aqueuse d'acide thioglycolique (1M, pH 9), et on laisse poser 15 mn. Les mèches sont alors lavées à l'eau et séchées pendant 20 mn à 60°C et utilisées immédiatement.

### Etape 2 : traitement avec le nitrate d'argent et réduction.

Les mèches réduites (1g) sont disposées chacune dans une gouttière appropriée. Pour chaque mèche, on applique 2 ml d'une solution aqueuse de nitrate d'argent (0,46 M) et on laisse poser 5 mn. On applique ensuite sur chaque mèche 1 ml d'une solution aqueuse de borohydrure de sodium (0,92M) et on laisse poser 15 mn. les mèches sont alors lavées à l'eau et séchées pendant 20 mn à 60°C.

On obtient les mêmes résultats visuels que dans l'exemple 1.

### Exemple 3 : démaquillage du gainage métallique d'argent

Une mèche de cheveux légèrement éclaircis, traitée selon l'exemple 1, est trempée dans une solution d'eau oxygénée à 30 volumes (9% en poids). La teinte cuivrée de la mèche disparaît instantanément et la mèche retrouve sa couleur originale avec un fort dégagement gazeux. Grâce à ce traitement, le gainage métallique présent sur le cheveu est donc démaquillé.

### Exemple 4 : greffage de 1-octadécanethiol sur mèches de cheveux sensibilisés gainées par un dépôt métallique d'argent

Les mèches (1g) traitées selon l'exemple 1 sont disposées chacune dans une gouttière appropriée. On applique sur chaque mèche 5 ml d'une solution aqueuse de NaOH à 1mM puis 5 ml d'une solution de thiol à 0,2 mM. La solution de thiol est obtenue en dissolvant 0,654g de 1-octadécanethiol dans l'éthanol (llitre), puis cette solution est diluée 11 fois dans l'éthanol. On laisse incuber une nuit à température ambiante. Les mèches sont ensuite essorées puis rincées avec 20 ml d'une solution EtOH/NaOH_{aq} 1mM, 50/50 (v/v). Finalement, les mèches sont rincées à l'eau et séchées pendant 20 mn à 60°C, puis lavées 5 fois avec un shampooing standard.

Afin de comparer les mèches traitées avec le 1-octadécanethiol avec les mèches non traitées gainées selon l'exemple 1, on utilise un test sensoriel.
- Qualité du toucher (de 0 à 5, 0=très mauvaise, 5=très bonne)
- Douceur (de 0 à 5, 0=très mauvaise, 5=très bonne)
- Propreté des doigts après toucher (de 0 à 5, 0=très mauvaise, 5=très bonne)

Les résultats sont rassemblés dans le tableau suivant :

| Mèches | Qualité toucher | Douceur | Propreté des doigts |
|---|---|---|---|
| Mèches traitées | 4 | 4,5 | 4 |
| Mèches non traitées (selon l'exemple 1) | 3 | 2,5 | 4 |

Les mèches traitées avec le 1-octadécanethiol et lavées présentent donc une qualité du toucher et une douceur supérieures aux mèches non traitées selon l'exemple 1.

## Revendications

1. Procédé de traitement cosmétique des fibres kératiniques, **caractérisé en ce qu'**il consiste à procéder à la réduction, dans un milieu cosmétiquement acceptable, d'au moins un sel métallique en présence d'au moins un agent réducteur, directement sur les fibres pour former in situ le métal natif correspondant, et **en ce que** postérieurement à la formation du métal natif correspondant, on applique sur les fibres kératiniques une composition contenant an moins un composé choisi parmi les thiols, les thiolates, les disulfures, les thioéthers, les xanthates, les thiocarbamates, les thiosulfates et les thiolactones.

2. Procédé selon la revendication 1, **caractérisé en ce que** le métal du sel métallique est choisi parmi l'aluminium, les métaux alcalins, les métaux alcalinoterreux, les métaux de transition, les métaux de terres rares et leurs alliages.

3. Procédé selon la revendication 1 ou 2, **caractérisé en ce que** le métal est choisi parmi l'aluminium, le cuivre, l'argent, l'or, l'indium, le fer, le platine, le nickel, le molybdène, le titane, le tungstène, l'antimoine, le palladium, le zinc, l'étain et leurs alliages.

4. Procédé selon l'une quelconque des revendications précédentes, **caractérisé en ce que** le sel métallique est un sel organique ou minéral, acide ou basique.

5. Procédé selon l'une quelconque des revendications précédentes, **caractérisé en ce que** le sel métallique est choisi parmi les nitrates, sulfates, carbonates, halogénures, bromates, phosphates et sulfonates.

6. Procédé selon l'une quelconque des revendications précédentes, **caractérisé en ce que** le sel métallique est choisi parmi AgNO₃, AgBrO₃, Ag₂SO₄, AgBr, Ag₂CO₃, AgCl, AgF, AgI, HAuCl₄, AuBr₃, AuCl₃, K₂PdCl₄, CuCl, CuBr, CuSO₄ et Na₂PtCl₆ et leurs mélanges.

7. Procédé selon l'une quelconque des revendications précédentes, **caractérisé en ce que** le sel métallique est choisi parmi HAuCl₄, AgNO₃, K₂PdCl₄, et Na₂PtCl₆ et leurs mélanges.

8. Procédé selon l'une quelconque des revendications précédentes, **caractérisé en ce que** l'agent réducteur est choisi parmi les réducteurs minéraux ou organiques.

9. Procédé selon l'une quelconque des revendications précédentes, **caractérisé en ce que** l'agent réducteur est choisi parmi NaBH₄, KBH₄, BH₃, NADPH, NADH, H₂, 9-BBN (9 borabicyclo[[3.3.1]nonane), LiAlH₄, NaAlEt₂H₂, Na₂S₂O₃, le diisoamylborane, l'acide ascorbique et les composés cycliques ou aliphatiques contenant le groupe chimique enediol - (OH)C=C(OH)- incluant les formes isomériques, leurs sels et leurs esters, l'hydroquinone, la benzoquinone, NaBH(OAc)₃, l'acide formamidine sulfinique (FASA), l'acide N-phénylformadimine sulfinique, les phosphines, NaBH₃CN, Na₂S₂O₄, BMS (bismercaptoéthylsulfone), DTT (dithiothréitol), DMH (N,N'diméthyl-N,N'-bis(mercaptoacétyl)hydrazine), les thiols, les thiolates, les citrates, sulfites, bisulfites, monopersulfates et bisulfates de métaux alcalins, les enzymes (comme par exemple la protéine disulfure isomérase ou la thioredoxine), et les sulfinates.

10. Procédé selon l'une quelconque des revendications précédentes, **caractérisé en ce que** le ou les sels métalliques et le ou les agents réducteurs sont présents à une concentration comprise entre 0,0001% et 50%, de préférence comprise entre 0,01% et 10%, et encore de préférence entre 0,05% et 5%, sur la base du milieu cosmétiquement acceptable.

11. Procédé selon l'une quelconque des revendications précédentes, **caractérisé en ce que** le milieu cosmétiquement acceptable est constitué par de l'eau et/ou un solvant organique choisi dans le groupe comprenant les alcools en C₁-C₄ tels que l'éthanol ou l'isopropanol, les alcanes en C₅-C₁₀, l'acétone, la méthyléthylcétone, l'acétate de méthyle, l'acétate de butyle, l'acétate d'éthyle, le diméthoxyéthane, le diéthoxyéthane et leurs mélanges

12. Procédé selon l'une quelconque des revendications précédentes, **caractérisé en ce que** le milieu cosmétiquement acceptable comprend en outre des additifs cosmétiques usuels choisis notamment parmi les agents adhésifs, les agents épaississants, les adoucissants, les agents anti-mousse, les filtres solaires, les agents antiperspirants, les agents acidifiants, les agents hydratants, les agents alcalinisants, les colorants, les pigments, les parfums, les conservateurs, les tensioactifs anioniques, cationiques, non ioniques ou amphotères, les polymères fixants, notamment les polymères fixants non ioniques, cationiques, amphotères, les polymères non fixants, les silicones volatiles ou non, les huiles végétales, animales, minérales ou de synthèse, les protéines et les vitamines, les polyols et leurs mélanges.

13. Procédé selon l'une quelconque des revendications précédentes, **caractérisé en ce que** :
- on applique sur les fibres kératiniques une composition contenant au moins un sel métallique, dans un milieu cosmétiquement acceptable,
- après un temps de pose allant de 30 s à 30 mn, et un éventuel rinçage, on applique une composition contenant au moins un agent réducteur, dans un milieu cosmétiquement acceptable, sous l'action éventuelle de la chaleur, puis
- on applique sur les fibres kératiniques une composition contenant au moins un composé choisi parmi les thiols, les thiolates, les disulfures, les thioéthers les xanthates, les thiocarbamates, les thiosulfates et les thiolactones,
- on procède au rinçage après un nouveau temps de pose de 30 s à 30 mn, sous l'action éventuelle de la chaleur.

14. Procédé selon la revendication 13, **caractérisé en ce que** préalablement à l'application sur les fibres kératiniques de la composition contenant au moins un sel métallique, on réduit les fibres kératiniques à l'aide d'un agent réducteur tel que défini dans la revendication 9.

15. Procédé selon la revendication 14, **caractérisé en ce que** la réduction préalable est effectuée avec l'acide thioglycolique.

16. Procédé selon la revendication 13, **caractérisé en ce que** préalablement à l'application sur les fibres kératiniques de la composition contenant au moins un sel métallique, on enduit les fibres kératiniques d'un polymère ayant une affinité pour la surface comme les polyamines ou les dérivés de polysaccharides.

17. Procédé selon la revendication 13, **caractérisé en ce que** préalablement à l'application sur les fibres kératiniques de la composition contenant au moins un sel métallique, on fait subir aux fibres kératiniques un traitement cosmétique classique comme une coloration d'oxydation, une permanente, une décoloration, un shampooing ou une application de produit de coiffage.

## Patentansprüche

1. Verfahren zur kosmetischen Behandlung von Keratinfasern, **dadurch gekennzeichnet, dass** es darin besteht, in einem kosmetisch akzeptablen Medium mindestens ein Metallsalz in Gegenwart mindestens eines Reduktionsmittels direkt auf den Keratinfasern zu reduzieren, um in situ das entsprechende elementare Metall zu bilden, und **dadurch**, dass nach der Bildung des entsprechenden elementaren Metalls auf die Keratinfasern eine Zusammensetzung aufgetragen wird, die mindestens eine Verbindung enthält, die unter den Thiolen, Thiolaten, Disulfiden, Thioethern, Xanthogenaten, Thiocarbamaten, Thiosulfaten und Thiolactonen ausgewählt ist.

2. Verfahren nach Anspruch 1, **dadurch gekennzeichnet, dass** das Metall des Metallsalzes unter Aluminium, Alkalimetallen, Erdalkalimetallen, Übergangsmetallen, Seltenerdmetallen und deren Legierungen ausgewählt ist.

3. Verfahren nach Anspruch 1 oder 2, **dadurch gekennzeichnet, dass** das Metall unter Aluminium, Kupfer, Silber, Gold, Indium, Eisen, Platin, Nickel, Molybdän, Titan, Wolfram, Antimon, Palladium, Zink, Zinn und deren Legierungen ausgewählt ist.

4. Verfahren nach einem der vorhergehenden Ansprüche, **dadurch gekennzeichnet, dass** das Metallsalz ein anorganisches oder organisches, saures oder basisches Salz ist.

5. Verfahren nach einem der vorhergehenden Ansprüche, **dadurch gekennzeichnet, dass** das Metallsalz unter den Nitraten, Sulfaten, Carbonaten, Halogeniden, Bromaten, Phosphaten und Sulfaten ausgewählt ist.

6. Verfahren nach einem der vorhergehenden Ansprüche, **dadurch gekennzeichnet, dass** das Metallsalz unter AgNO₃, AgBrO₃, Ag₂SO₄, AgBr, Ag₂CO₃, AgCl, AgF, AgI, HAuCl₄, AuBr₃, AuCl₃, K₂PdCl₄, CuCl, CuBr, CuSO₄ und Na₂PtCl₆ und deren Gemischen ausgewählt ist.

7. Verfahren nach einem der vorhergehenden Ansprüche, **dadurch gekennzeichnet, dass** das Metallsalz unter HAuCl₄, AgNO₃, K₂PdCl₄ und Na₂PtCl₆ und deren Gemischen ausgewählt ist.

8. Verfahren nach einem der vorhergehenden Ansprüche, **dadurch gekennzeichnet, dass** das Reduktionsmittel unter den anorganischen oder organischen Reduktionsmitteln ausgewählt ist.

9. Verfahren nach einem der vorhergehenden Ansprüche, **dadurch gekennzeichnet, dass** das Reduktionsmittel unter NaBH₄, KBH₄, BH₃, NADPH, NADH, H₂, 9-BBN (9-Borabicyclo[3.3.1]nonan, LiAlH₄, NaAlEt₂H₂, Na₂S₂O₃, Diisoamylboran, Ascorbinsäure und cyclischen oder aliphatischen Verbindungen, die die chemische Endiolgruppe (OH)C=C(OH)- enthalten, einschließlich der isomeren Formen, ihren Salzen und ihren Estern, Hydrochinon, Benzochinon, NaBH(OAc)₃, Formamidinsulfinsäure (FASA), N-Phenylformadiminsulfinsäure, Phosphinen, NaBH₃CN, Na₂S₂O₄, BMS (Bismercaptoethylsulfon), DTT (Dithiothreit), DMH (N,N'-Dimethyl-N,N'-bis(mercaptoacetyl)hydrazin), Thiolen, Thiolaten, Citraten, Sulfiten, Bisulfiten, Alkalimetallmonopersulfaten und Alkalimetallbisulfaten, Enzymen (wie beispielsweise Protein-Disulfid-Isomerase oder Thioredoxin) und Sulfinaten ausgewählt ist.

10. Verfahren nach einem der vorhergehenden Ansprüche, **dadurch gekennzeichnet, dass** das Metallsalz oder die Metallsalze und das Reduktionsmittel oder die Reduktionsmittel in einer Konzentration von 0,0001 bis 50 %, vorzugsweise 0,01 bis 10 % und noch bevorzugter 0,05 bis 5 %, auf der Basis des kosmetisch akzeptablen Mediums, enthalten sind.

11. Verfahren nach einem der vorhergehenden Ansprüche, **dadurch gekennzeichnet, dass** das kosmetisch akzeptable Medium aus Wasser und/oder einem organischen Lösungsmittel besteht, das unter den C₁₋₄-Alkoholen, wie Ethanol oder Isopropanol, C₅₋₁₀₋Alkanen, Aceton, Methylethylketon, Methylacetat, Butylacetat, Ethylacetat, Dimethoxyethan, Diethoxyethan und deren Gemischen ausgewählt ist.

12. Verfahren nach einem der vorhergehenden Ansprüche, **dadurch gekennzeichnet, dass** das kosmetisch akzeptable Medium ferner mindestens einen üblichen kosmetischen Zusatzstoff enthält, der unter den adhäsiven Stoffen, Verdickungsmitteln, beruhigenden Stoffen, Schaumverhütungsmitteln, Sonnenschutzfiltern, Antiperspirantien, Ansäuerungsmitteln, Hydratisierungsmitteln, Alkalisierungsmitteln, Farbmitteln, Pigmenten, Parfums, Konservierungsmitteln, anionischen, kationischen, nichtionischen oder amphoteren grenzflächenaktiven Stoffen, festigenden Polymeren und insbesondere nichtionischen, kationischen, amphoteren festigenden Polymeren, nichtfestigenden Polymeren, flüchtigen oder nichtflüchtigen Siliconen, pflanzlichen Ölen, tierischen Ölen, Mineralölen oder synthetischen Ölen, Proteinen und Vitaminen, Polyolen und deren Gemischen ausgewählt sind.

13. Verfahren nach einem der vorhergehenden Ansprüche, **dadurch gekennzeichnet, dass**:
- auf die Keratinfasern eine Zusammensetzung aufgetragen wird, die in einem kosmetisch akzeptablen Medium mindestens ein Metallsalz enthält,
- nach einer Einwirkzeit von 30 s bis 30 min gegebenenfalls gespült wird, und dann eine Zusammensetzung, die in einem kosmetisch akzeptablen Medium mindestens ein Reduktionsmittel enthält, gegebenenfalls unter Einwirkung von Wärme aufgebracht wird, und
- auf die Keratinfasern eine Zusammensetzung aufgetragen wird, die mindestens eine Verbindung enthält, die unter den Thiolen, Thiolaten, Disulfiden, Thioethern, Xanthogenaten, Thiocarbamaten, Thiosulfaten und Thiolactonen ausgewählt ist,
- und nach einer weiteren Einwirkzeit von 30 s bis 30 min gegebenenfalls unter Einwirkung von Wärme gespült wird.

14. Verfahren nach Anspruch 13, **dadurch gekennzeichnet, dass** die Keratinfasern, bevor die Zusammensetzung, die mindestens ein Metallsalz enthält, auf die Keratinfasern aufgetragen wird, mit einem Reduktionsmittel reduziert werden, wie es in Anspruch 9 definiert ist.

15. Verfahren nach Anspruch 14, **dadurch gekennzeichnet, dass** die vorhergehende Reduktion mit Thioglykolsäure durchgeführt wird.

16. Verfahren nach Anspruch 13, **dadurch gekennzeichnet, dass** die Keratinfasern, bevor die Zusammensetzung, die mindestens ein Metallsalz enthält, auf die Keratinfasern aufgetragen wird, mit einem Polymer beschichtet werden, das eine Affinität für die Oberfläche besitzt, wie Polyamine oder Polysaccharidderivate.

17. Verfahren nach Anspruch 13, **dadurch gekennzeichnet, dass** an den Keratinfasern, bevor die Zusammensetzung, die mindestens ein Metallsalz enthält, auf die Keratinfasern aufgetragen wird, eine herkömmliche kosmetische Behandlung durchgeführt wird, wie beispielsweise eine oxidative Färbung, eine permanente Verformung, eine Entfärbung, eine Haarwäsche oder das Aufbringen eines Produktes für die Frisurengestaltung.

## Claims

1. Cosmetic treatment process for keratin fibres, **characterized in that** it consists in reducing, in a cosmetically acceptable medium, at least one metal salt in the presence of at least one reducing agent, directly on the keratin fibres to form the corresponding native metal in situ, and **in that**, subsequent to the formation of the corresponding native metal, a composition containing at least one compound chosen from thiols, thiolates, disulphides, thioethers, xanthates, thiocarbamates, thiosulphates and thiolactones is applied to the keratin fibres.

2. Process according to Claim 1, **characterized in that** the metal of the metal salt is chosen from aluminium, alkali metals, alkaline-earth metals, transition metals, rare-earth metals and alloys thereof.

3. Process according to Claim 1 or 2, **characterized in that** the metal is chosen from aluminium, copper, silver, gold, indium, iron, platinum, nickel, molybdenum, titanium, tungsten, antimony, palladium, zinc and tin, and alloys thereof.

4. Process according to any one of the preceding claims, **characterized in that** the metal salt is an organic or mineral, acidic or basic salt.

5. Process according to any one of the preceding claims, **characterized in that** the metal salt is chosen from nitrates, sulphates, carbonates, halides, bromates, phosphates and sulphonates.

6. Process according to any one of the preceding claims, **characterized in that** the metal salt is chosen from AgNO₃, AgBrO₃, Ag₂SO₄, AgBr, Ag₂CO₃, AgCl, AgF, AgI, HAuCl₄, AuBr₃, AuCl₃, K₂PdCl₄, CuCl, CuBr, CuSO₄ and Na₂PtCl₆, and mixtures thereof.

7. Process according to any one of the preceding claims, **characterized in that** the metal salt is chosen from HAuCl₄, AgNO₃, K₂PdCl₄ and Na₂PtCl₆, and mixtures thereof.

8. Process according to any one of the preceding claims, **characterized in that** the reducing agent is chosen from mineral or organic reducing agents.

9. Process according to any one of the preceding claims, **characterized in that** the reducing agent is chosen from NaBH₄, KBH₄, BH₃, NADPH, NADH, H₂, 9-BBN (9-borabicyclo[3.3.1]nonane), LiAlH₄, NaAlEt₂H₂, Na₂S₂O₃, diisoamylborane, ascorbic acid and cyclic or aliphatic compounds containing the enediol chemical group -(OH)C=C(OH)- including the isomeric forms, salts thereof and esters thereof, hydroquinone, benzoquinone, NaBH(OAc)₃, formamidinesulphinic acid (FASA), N-phenylformamidinesulphinic acid, phosphines, NaBH₃CN, Na₂S₂O₄, BMS (bismercaptoethyl sulphone), DTT (dithiothreitol), DMH (N,N'-dimethyl-N,N'-bis(mercaptoacetyl)hydrazine), thiols, thiolates, citrates, sulphites, bisulphites, monopersulphates and bisulphates of alkali metals, enzymes (such as, for example, the protein disulphide isomerase or thioredoxin), and sulphinates.

10. Process according to any one of the preceding claims, **characterized in that** the metal salt(s) and the reducing agent(s) are present at a concentration of between 0.0001% and 50%, preferably between 0.01% and 10% and more preferably between 0.05% and 5%, relative to the cosmetically acceptable medium.

11. Process according to any one of the preceding claims, **characterized in that** the cosmetically acceptable medium consists of water and/or an organic solvent chosen from the group comprising C₁-C₄ alcohols such as ethanol or isopropanol, C₅-C₁₀ alkanes, acetone, methyl ethyl ketone, methyl acetate, butyl acetate, ethyl acetate, dimethoxyethane, diethoxyethane, and mixtures thereof.

12. Process according to any one of the preceding claims, **characterized in that** the cosmetically acceptable medium also comprises common cosmetic additives chosen especially from adhesives, thickeners, softeners, antifoams, sunscreens, antiperspirants, acidifying agents, moisturizers, basifying agents, colorants, pigments, fragrances, preserving agents, anionic, cationic, nonionic or amphoteric surfactants, fixing polymers, especially nonionic, cationic or amphoteric fixing polymers, non-fixing polymers, volatile or non-volatile silicones, plant, animal, mineral or synthetic oils, proteins, vitamins, polyols, and mixtures thereof.

13. Process according to any one of the preceding claims, **characterized in that**:
- a composition containing at least one metal salt, in a cosmetically acceptable medium, is applied to the keratin fibres,
- after an action time ranging from 30 seconds to 30 minutes, and optional rinsing, a composition containing at least one reducing agent, in a cosmetically acceptable medium, is applied under the optional action of heat, and then
- a composition containing at least one compound chosen from thiols, thiolates, disulphides, thioethers, xanthates, thiocarbamates, thiosulphates and thiolactones is applied to the keratin fibres,
- after a further action time of 30 seconds to 30 minutes, under the optional action of heat, the fibres are rinsed.

14. Process according to Claim 13, **characterized in that**, prior to applying the composition containing at least one metal salt to the keratin fibres, the keratin fibres are reduced using a reducing agent as defined in Claim 9.

15. Process according to Claim 14, **characterized in that** the prior reduction is performed with thioglycolic acid.

16. Process according to Claim 13, **characterized in that**, prior to applying the composition containing at least one metal salt to the keratin fibres, the keratin fibres are coated with a polymer that has affinity for the surface, for instance polyamines or polysaccharide derivatives.

17. Process according to Claim 13, **characterized in that**, prior to applying the composition containing at least one metal salt to the keratin fibres, the keratin fibres are subjected to a standard cosmetic treatment, for instance an oxidation-dyeing, permanent-wave or bleaching operation, shampooing or an application of styling product.
